## Europäisches Patentamt

## European Patent Office

## Office européen des brevets

(19)

(11) Veröffentlichungsnummer: **0 067 931**
**B1**

(12)

# EUROPÄISCHE PATENTSCHRIFT

(45) Veröffentlichungstag der Patentschrift:
02.09.87

(51) Int. Cl.⁴: **G 01 N 27/56**

(21) Anmeldenummer: **82102890.9**

(22) Anmeldetag: **05.04.82**

(54) Verfahren und Vorrichtung zur Überwachung und Kalibrierung von Grenzstromsonden.

(30) Priorität: **16.04.81 DE 3115404**

(43) Veröffentlichungstag der Anmeldung:
**29.12.82 Patentblatt 82/52**

(45) Bekanntmachung des Hinweises auf die Patenterteilung:
**02.09.87 Patentblatt 87/36**

(84) Benannte Vertragsstaaten:
**DE FR GB IT**

(56) Entgegenhaltungen:
DE-A-2 606 727
DE-A-2 733 524
DE-A-2 735 434
FR-A-1 375 183
FR-A-2 291 480
US-A-3 938 075
US-A-4 031 747
US-A-4 151 738

(73) Patentinhaber: **ROBERT BOSCH GMBH, Postfach
50, D-7000 Stuttgart 1 (DE)**

(72) Erfinder: **Dietz, Hermann, Dr., Steinbeissstrasse
48, D-7016 Gerlingen (DE)**
Erfinder: **Grob, Ferdinand, Dipl.- Ing., Friedrich-
Schelling Weg 8, D-7122 Besigheim (DE)**
Erfinder: **Müller, Klaus, Dr., Schillerstrasse41,
D-7146 Tamm (DE)**
Erfinder: **Reber, Harald, Dr., Talstrasse 4, D-7016
Gerlingen (DE)**

## Beschreibung

### Stand der Technik

Die Erfindung geht aus von einem Verfahren und Kalibierung von Grenzstromsonden nach der Gattung des Anspruchs 1 und einer Vorrichtung nach der Gattung des Anspruchs 4.

Aus der FR-A- 22 91 480 ist eine Korrektureinrichtung zur selbsttätigen Driftkorrektur eines Analysengerätes bekannt, bei der der Verstärkungsgrad einer Verstärkeranordnung so beeinflußt wird daß beim Zuführen eines ersten Kontrollmediums zu einer Meßeinrichtung für die Definition eines Meßbereichsanfangspunktes und hernach bei Zuführen eines zweiten Kontrollmediums für die Definition eines Meßbereichsendpunktes die Ausgangsspannungen der Verstärkeranordnung genau vorgegebenen Sollwerten entsprechen. Diese Einrichtung sieht also eine derartige automatische Eichung einer Meßanordnung auf zwei Referenzmedien vor, daß ein Meßbereich zwischen zwei eichbare Grenzwerte "eingepaßt" wird. Sie sieht aber nicht die Überwachung und Kalibrierung eines Gebers in der Form einer toleranzbehafteten Sonde vor, wobei aus der Beaufschlagung mit nur einem Referenzmedium eine Aussage über eine gegebene oder nicht mehr gegebene Funktionsfähigkeit der Sonde ableitbar ist. Um die Meßbereichseinpassung zu leisten, muß dem Eingang des beschriebenen Differenzverstärkers noch eine veränderbare Spannung zugeführt werden; aus diesem Grund wird bei dieser bekannten Einrichtung das Meßergebnis verfälscht durch eine nur unzureichend mögliche Offsetspannungskorrektur der Verstärkeranordnung.

Aus der DE-A- 26 08 727 ist ein Verfahren zur Partialdruckmessung von Gasen bekannt, das zur Kompensation eines sich ändernden Ausgangssignals eines Meßfühlers ebenfalls eine Veränderug der Verstärkung eines Meßverstärkers vorsieht. Die Kalibrierung nach dem beschriebenen Verfahren weist denselben Nachteil einer sich verstellenden Offsetspannung - begünstigt durch den sich regelmäßig ändernden Eingangswiderstand des Verstärkers in Abhängigkeit von der eingestellten Verstärkung - auf. Die Funktion einer Überwachung der Gaselektrode zur Partialdruckmessung ist hingegen nicht realisiert.

Die FR-A- 13 75 183 beschreibt in allgemeiner Form die Einsetzbarkeit von Rechenanlagen zur Kontrolle industrieller Prozesse. Eine technische Lehre zur Überwachung einer Grenzstromsonde, insbesondere einer Sauerstoff-Grenzstromsonde, ist dieser Schrift jedoch nicht zu entnehmen.

## Vorteile der Erfindung

Das erfindungsgemäße Verfahren bzw. die erfindungsgemäße Vorrichtung mit den Merkmalen des Anspruches 1 bzw. 4 hat demgegenüber den Vorteil, daß eine Grenzstromsonde im eingebauten Zustand mit der dazugehörigen Meß- oder Regelschaltung überwachbar ist und eine Nacheichung oder Fehleranzeige erfolgt. Als weiterer Vorteil ist anzusehen, daß durch die Nacheichung auch Sonden verwendbar sind, die nicht im Toleranzbereich einer ordnungsgemäß arbeitenden Sonde liegen. Fehlmessungen mit der Grenzstromsonde werden somit vermieden.

Durch die in den abhängigen Ansprüchen aufgeführten Maßnahmen sind vorteilhafte Weiterbildungen und Verbesserungen des im Anspruch 1 angegebenen Verfahrens bzw. der im Anspruch 4 angegebenen Vorrichtung möglich. Besonders vorteilhaft ist es, wenn der ermittelte Sondenstromsollwert zur Festlegung einer Meßkennlinie dient. Durch diese Maßnahme ist es möglich, auch nichtlineare Kennlinien festzulegen. Es ist auch vorteilhaft, bei einer so großen Abweichung vom Sondenstromsollwert eine Abschaltung der Sonde vorzunehmen, da dann damit zu rechnen ist, daß die Sonde insgesamt defekt ist. Als Normgas hat sich Luft besonders einfach und vorteilhaft erwiesen. Es ist weiterhin günstig, den letzten bei Normgas ermittelten Sondenstrom zu speichern und zur Kennlinienkorrektur der Sondenkennlinie zu verwenden. Dies ergibt eine besonders einfache Ausgestaltung der Erfindung. Weiterhin ist es günstig, mehrere Sondenstromwerte zu speichern und über den letzten Sondenstromwert hinaus zu extrapolieren. Hierdurch sind frühzeitig Fehler an der Sonde erkennbar, so daß sie vor ihrem Ausfall ausgetauscht werden kann. Weiterhin ist es vorteilhaft, die Versorgungsspannung der Sonde um einen Kennwert zu variieren und die Stromänderung dabei zu messen. Durch diese Maßnahme kann erkannt werden, wenn sich die Sondenkennlinie stark ändert. Um Überschwingungen zu vermeiden, ist es günstig, die Spannungsänderung mit einer Frequenz von < 1 Hz ablaufen zu lassen. Vorteilhafterweise erfolgt die Bestimmung des Grenzstromsollwertes kurz vor dem Einschalten des Gerätes, beispielsweise der Brennkraftmaschine, da insbesondere nach längerer Standzeit die Sonde mit Luft umspült ist. Eine weitere Möglichkeit ist die Bestimmung des Sondenstromsollwertes nach einer vorgegebenen Zeit nach dem Ausschalten des Gerätes.

Die Nacheichung läßt sich beispielsweise mit einer einstellbaren Gleichspannungsquelle vornehmen, die über eine Regelschaltung verstellbar ist und zur Aufnahme des Sondenstromsollwertes dient. Die Gleichspannungsquelle kann dabei vorteilhafterweise als Widerstand ausgebildet sein, der von einem Motor durch die Regeleinrichtung verstellbar ist. Ein solcher einstellbarer Widerstand kann dabei die Funktion eines Sondenstromsollwertspeichers übernehmen. Eine einfache Ausgestaltung der Regelschaltung ergibt sich dadurch, daß der Meßwiderstand des Sondenstromes umschaltbar ist. Durch diese Maßnahme kann eine Regelschaltung sowohl den Betrieb als auch die Überprüfung der Sonde durchführen. Der einstellbare Widerstand weist vorteilhafterweise Anschläge auf, bei deren Berühren durch den Schleifer die Regelvorrichtung abgeschaltet wird. Hierdurch können große Abweichungen des Sondenstromsollwertes erkannt werden.

Vorteilhaft kann eine Vorrichtung zur Überwachung und Kalibirierung von Sauerstoffsonden mittels einer Recheneinrichtung, insbesondere einem Mikrocomputer realisiert werden, welcher sowohl die Regelung besorgt als auch zumindest einen Speicher aufweist, indem die Grenzstromsollwerte abspeicherbar sind. In Abhängigkeit vom Sondenstromsollwert sind beliebige, im Speicher abgelegte Kennlinien abrufbar. Dadurch sind beliebige Sonden an die Schaltungsanordnung anpaßbar. Mittels der Recheneinrichtung ist auch die Gleichspannung der Gleichspannungsquelle der Sauerstoffsonde änderbar, um die Stromänderung der Sauerstoffsonde zu erfassen. Eine Extrapolation ist besonders einfach möglich, wenn mindestens zwei frühere Sondenstromsollwerte im Speicher abgelegt sind. Hierdurch ist frühzeitig der Ausfall der Sauerstoffsonde erkennbar. Weiterhin ist es vorteilhaft, beim Überschreiten oder Unterschreiten vorgegebener Sondenstromwerte oder beim Überschreiten eines vorgegebenen Wertes zwischen zwei Sollwertmessungen einen Fehlalarm durch die Recheneinrichtung auszulösen oder die Recheneinrichtung auf ein Notprogramm umzuschalten. Dadurch wird erreicht, daß ein Schaden der Sonde rechtzeitig erkannt wird, jedoch ein Stillegen der Anlage nicht erforderlich ist. Als Recheneinrichtung eignet sich besonders ein Einchipmikrocomputer. Die Vorrichtungen sind besonders vorteilhaft bei Abgasmessungen in Heizanlagen oder bei Kraftfahrzeugen mit Verbrennungsmotor einsetzbar, da bei diesen Anlagen üblicherweise Luft als Normgas zur Sauerstoffsonde gelangen kann.

## Zeichnung

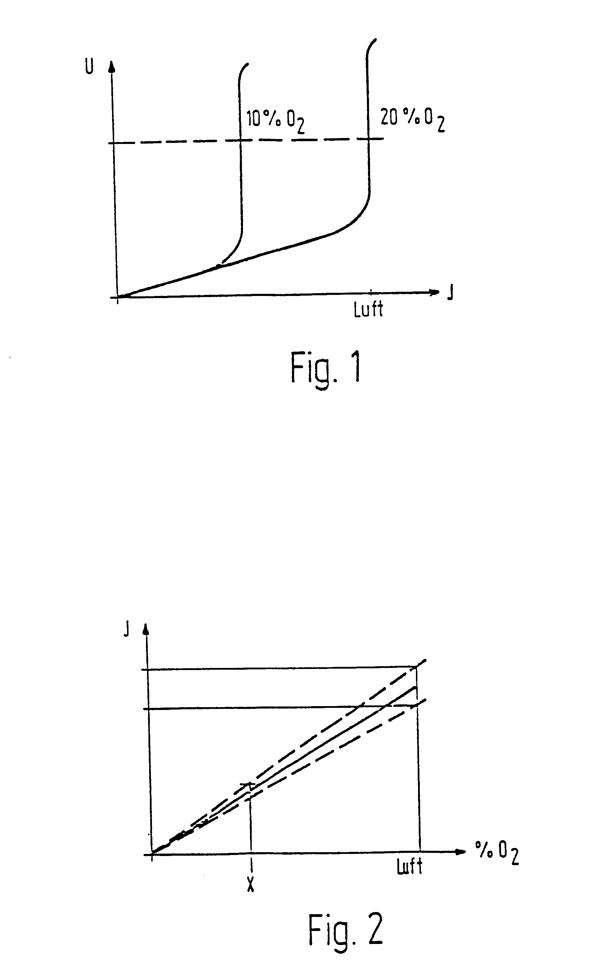

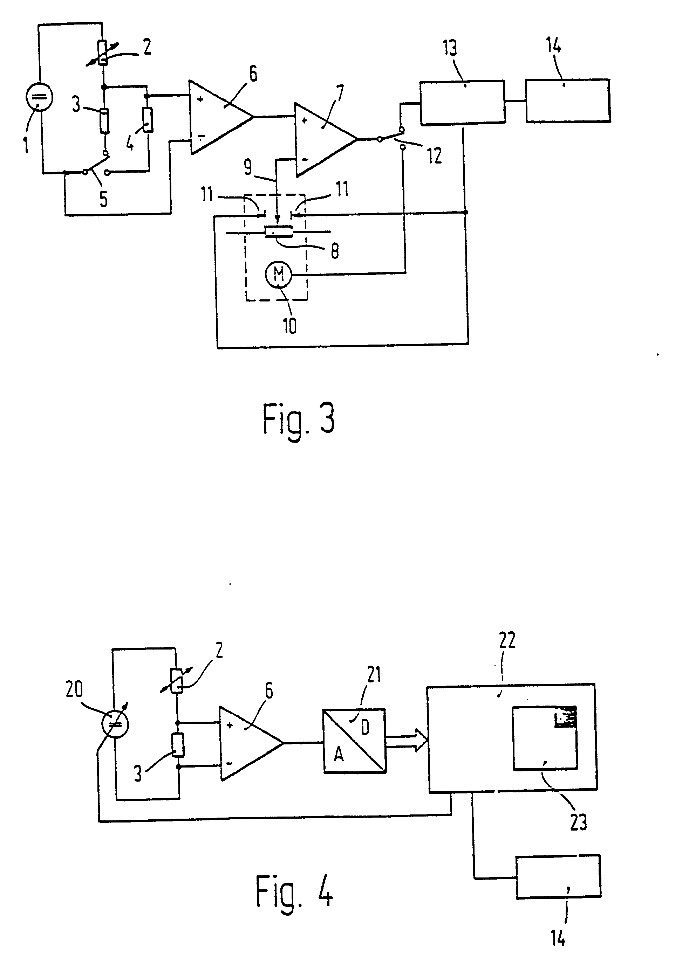

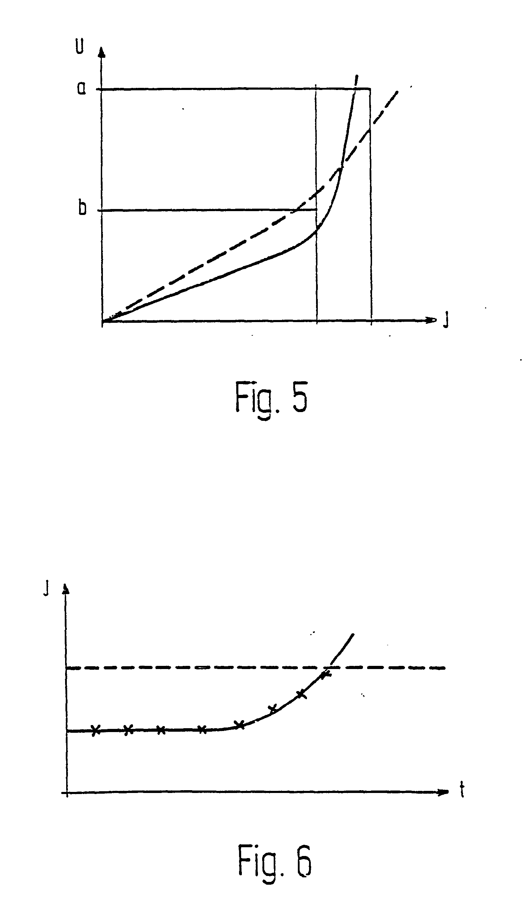

Ein Ausführungsbeispiel der Erfindung ist in der Zeichnung dargestellt und in der nachfolgenden Beschreibng näher erläutert. Es zeigen zunächst Figur 1 die Kennlinien einer Grenzstromsonde bei unterschiedlichem Sauerstoffgehalt, Figur 2 eine Kennlinie des Sondenstromes in Abhängigkeit vom Sauerstoffgehalt und Figur 3 eine figürliche Erläuterung einer Möglichkeit zur Überwachung und Kalibrierung von Sauerstoffsonden. Figur 4 zeigt das aus Figur 3 entwickelte erfindungsgemäße Ausführungsbeispiel, Figur 5 einen Ausschnitt einer Grenzstromsondenkennlinie nach Figur 1 und Figur 6 die zeitliche Änderung des Grenzstromsondenstromes.

## Beschreibung des Ausführungsbeispiels

Die Luft-Kraftstoff-Zusammensetzung des Betriebsgemisches bei Brennkraftmaschinen oder die günstige Einstellung einer Heizungsanlage wird mit Hilfe einer Grenzstromsonde auf ein bestimmte Luftverhältnis geregelt. Es handelt sich dabei um eine Sauerstoffmeßsonde, die auf den Sauerstoffpartialdruck im Abgas der Brennkraftmaschine oder der Heizungsanlage anspricht. An die Elektroden einer solchen Sonde wird dabei eine Meßspannung angelegt, durch die ein Meßstrom aufgrund eines Sauerstoffionenflusses durch den Festelektrolytkörper der Sonde zustande kommt. Die Höhe des Meßstromes wird durch die Diffusionsgeschwindigkeit des Sauerstoffs begrenzt und ist abhängig von der Konzentration des Sauerstoffs in dem zu messenden Gas. Spannungsabweichungen der Meßspannung innerhalb eines bestimmten Bereiches haben im stationären Fall daher keine Auswirkung auf den Stromfluß, der einen durch die Diffusionsgeschwindigkeit begrenzten Stromwert beibehält. Die Kennlinie einer Sonde in Abhängigkeit vom Sauerstoffgehalt ist in Figur 1 dargestellt. Es ist erkennbar, daß bei Spannungsschwankungen im geringen Umfang eine Stromänderung bei der Sonde nicht auftritt. Die Abhängigkeit des Stromes, der durch die Grenzstromsonde fließt, in Abhängigkeit vom Sauerstoffgehalt ist in der Figur 2 als durchgezogene Linie dargestellt. Grenzstromsonden unterliegen mit der Zeit Veränderungen, die im Geber selbst auftreten oder durch die Einsatzbedingungen verursacht werden. Der Geber driftet dann, und die Veränderungen sind oft irreversibel. Aus diesem Grunde ist die Selbstüberwachung der Grenzstromsonde von Bedeutung, so daß eine Nachkalibrierung möglich ist, da insbesondere bei Grenzstromsonden eine Verschiebung des Arbeitspunktes erst zu einem späteren Zeitpunkt oder gar nicht erkannt wird. Verschmutzt beispielsweise die Sonde, verschiebt sich die Kennlinie, wie dies beispielsweise in Figur 2 gestrichelt dargestellt ist. Die Erfindung geht nun von dem Gedanken aus, daß die Grenzstromsonden in Heizungsanlagen und in Kraftfahrzeugen zumindest zeitweise der Umgebungsluft ausgesetzt ist, da Kraftfahrzeuge und Heizungsanlagen nur zeitweise in Betrieb sind. Die Luft hat den bekannten Sauerstoffanteil von 20,8 %. Bei abgeschaltetem Verbrennungsprozeß ist es daher möglich, eine Kalibrierung mit der Sonde vorzunehmen. In den meisten Fällen, wie beispielsweise bei Verschmutzung der Sonde, ändert sich, wie in Figur 2 dargestellt, nur die Steigung der Kennlinie, so daß ein Meßwert ausreicht, um die Kalibrierung vorzunehmen.

In Figur 3 ist eine besonders einfache Möglichkeit zur Kalibrierung und Überwachung einer Sauerstoff-Grenzstromsonde wiedergegeben. Eine Gleichspannungsquelle 1 ist mit einer Grenzstromsonde 2 verbunden. Der Grenzstromsonde 2 schließt sich ein Meßwiderstand 3 und ein Eichwiderstand 4 an, die mittels eines Umschalters 5 mit der Gleichspannungsquelle 1 in Verbindung stehen. Zwischen Grenzstromsonde 2 und den Widerständen 3 und 4 führt eine Leitung zum nicht invertierenden Eingang des Differenzverstärkers 6. Der invertierende Eingang des Differenzverstärkers 6 ist an den Umschalter 5 angeschlossen. Der Ausgang des Differenzverstärkers 6 führt zum nicht invertierenden Eingang des Differenzverstärkers 7. Der invertierende Eingang des Differenzverstärkers 7 steht mit einem Abgriff 9 eines Potentiometers 8 in Verbindung, das an eine Gleichspannungsquelle angeschlossen ist. Zwei Anschläge 11 begrenzen den Weg des Abgriffs 9 und geben ein Signal ab, wenn der Abgriff 11 an einen der Anschläge 11 stößt. Die Anschläge 11 wirken mittels Leitungen auf den Regler 13 ein. Der Abgriff 9 ist mittels eines Motors 10 antreibbar, der über einen Umschalter 12 mit dem Ausgang des Differenzverstärkers 7 in Verbindung steht. Ein weiterer Kontakt des Umschalters 12 führt zu einem Regler 13, der ein Luftmengenstellglied in einer Heizungsanlage 14 betätigt. Die Grenzstromsonde 2 mißt das Abgas der Heizungsanlage 14.

Im Arbeitsbetrieb sind die Umschalter 5 und 12 so geschaltet, daß die Spannung am Meßwiderstand 3 abgegriffen wird und das Ausgangssignal des Differenzverstärkers 7 an den Regler 13 gelangt. Schaltet die Heizungsanlage 14 aus, so wird in den Abgaskanal mittels eines nicht dargestellten Gebläses Frischluft geblasen. In vielen Fällen genügt es jedoch auch, eine gewisse Zeit zu warten, bis in den Abgaskanal Frischluft gelangt ist. Danach werden die Umschalter 5 und 12 betätigt. Der Eichwiderstand 4 ist so dimensioniert, daß an ihm die gleiche Spannung abfällt, wenn sich die Grenzstromsonde 2 in der Luft befindet, wie am Meßwiderstand 3, wenn sich die Grenzstromsonde 2 unter Betriebsbedingungen befindet. Diese Spannung wird im Differenzverstärker 6 verstärkt und mit einem früheren eingestellten Sollwert verglichen, der am invertierenden Eingang des Differenzverstärkers 7 anliegt. Haben sich die Daten der Grenzstromsonde 2 nicht geändert, so wird dieser Wert gleich sein, so daß kein Regelvorgang stattfindet. Wird jedoch ein zu niedriger oder zu hoher Wert gemessen, so bedeutet dies, daß die Grenzstromsonde unter Normalbedingungen einen zu hohen oder niedrigen Strom aufnimmt. Durch den Motor 10 wird nun der Abgriff 9 des Potentiometers 8 solange verstellt, bis die Anordnung im Gleichgewicht ist. Diese Änderung wirkt einfach wie eine Steigungsänderung der Geraden in Figur 2. Durch eine höhere oder niedrigere Spannung am Abgriff 9 ist daher der Fehler an der Grenzstromsonde automatisch abgeglichen. Am Meßpunkt x tritt bei einer entsprechenden Sauerstoffkonzentration der gleiche Strom auf. Sind die Abweichungen der Grenzstromsonde von ihren ursprünglichen Daten zu groß, gelangt der Abgriff 9 an einen der Anschläge 17. In diesem Fall wird der Regler 13 außer Betrieb gesetzt und ein Festwert als Referenzwert genommen. Soll bereits eine frühe Warnung erfolgen, ist es günstig, an beiden Seiten des Potentiometers jeweils zwei Anschläge anzubringen, wobei bei dem ersten Anschlag eine früher Ausfallanzeige ausgelöst wird, während erst im Bereich des zweiten Anschlages ein Abschalten des Reglers 13 erfolgt.

Dieses Schaltungsbeispiel zeigt eine besonders einfache Realisierungsmöglichkeit zur Kalibrierung der Grenzstromsonde. Durch geeignete Maßnahmen ist sicherzustellen, daß die Eichung nur erfolgt, wenn die Grenzstromsonde nur mit Luft umspült wird. Statt einer Luftumspülung ist es ebenfalls möglich, eine Bypass-Schaltung mit einem Luft-Bypass an der Sondeneinbaustelle vorzusehen. Statt Luft kann auch ein anderes definiertes Gasgemisch verwendet werden. Ist der Abgleichvorgang zu Ende, geht das System wieder in die Meßstellung zurück, d.h. die Schalter 5 und 12 werden umgeschaltet. Dies kann beispielsweise über ein Zeilglied geschehen, das solange in den Kalibrierzustand schaltet, wie dies üblicherweise zum Ausregeln des Sollwertes erforderlich ist.

Die Sollwertkorrektur gestaltet sich im Fall einer digitalen Regelung jedoch erheblich einfacher, wie dies erfindungsgemäß in Figur 4 dargestellt ist. Eine regelbare Gleichspannungsquelle 20 ist mit der Grenzstromsonde 2 verbunden. Über den Meßwiderstand 3, der mit der Gleichspannungsquelle 20 in Verbindung steht, wird der Stromkreis geschlossen. Der Differenzverstärker 6 greift die am Meßwiderstand 3 abfallende Spannung ab und leitet sie dem Analog-Digital-Wandler 27 zu. Der Analog-Digital-Wandler steht über eine Datenleitung mit dem Mikrocomupter 22 in Verbindung, dem die gemessene Spannung als digitales Signal zugeführt ist. Der Mikrocomputer 22 weist des weiteren einen Speicher 23 auf. Ein Ausgang des Mikrocomputers führt zum Luftmengenstellglied der Heizungsanlage 74. Ein weiterer Ausgang des Mikrocomputers 22 wirkt auf die Gleichspannungsquelle 20 ein.

Der gesamte Ablauf ist im wesentlichen der gleiche, wie dies bereits anhand der Figur 3 beschrieben worden ist.

Jedoch laufen alle Vorgänge rechnergesteuert ab. Das Sondensignal wird durch den Analog-Digital-Wandler 21 in ein digitales Signal umgewandelt, das mit dem Sollwert verglichen wird, der im Speicher 23 des Mikrocomputers 22 gespeichert ist. Eine entsprechende Steuerung der Heizungsanlage 14 erfolgt dann durch den Mikrocomputer 22. Der Mikrocomputer steuert auch die Umschaltung in den Kalibrierbetrieb, in dem er nach dem Einströmen von Luft in den Abgaskanal der Heizungsanlage 14 den Stromsollwert mißt und in ein entsprechendes Register schreibt. Im einfachsten Fall wird dabei der neue Meßwert in das gleiche Register geschrieben, in dem bereits zuvor der Sondenstromsollwert gespeichert ist. Zuvor wird der gemessene Wert mit ebenfalls in Registern abgelegten Grenzwerten verglichen, wobei beim Erreichen eines ersten Grenzwertes eine Fehlermeldung ausgelöst wird, während beim Erreichen eines zweiten Grenzwertes die Regeleinrichtung vollständig abgeschaltet wird oder aber vom Mikrocomputer ein Notprogramm abgewickelt wird.

Zur Trendverfolgung bietet sich eine Speicherkette an, in die der vorhergehende Sollwert eingeschoben wird. Erfolgt dieser Vorgang äquidistant, so kann der Trend direkt für die nächsten zwei bis drei Kalibrierintervalle angegeben werden, und zwar durch eine einfache Extrapolation aufgrund der beiden letzten Sollwerte oder durch das genauere Verfahren einer parabolischen Anpassung an die letzten drei Werte mit Bildung der Ableitung am letzten Sollwert und Extrapolation oder durch Berechnung einer Ausgleichskurve, die im allgemeinen exponentiell gegen eine Asymptote strebt. Diese drei erwähnten Verfahren zur Trendverfolgung können gestuft angewendet werden, insbesondere, wenn noch nicht genügend Meßwerte zur Verfügung stehen. Ein Beispiel der Trendverfolgung ist in Fig. 6 dargestellt. Durch die Trendverfolgung ist es bereits frühzeitig möglich, vor dem Erreichen eines kritischen Zustandes einen Alarm auszulösen, der den Sondenwechsel ermöglicht, ohne daß die Heizungsanlage deswegen abgeschaltet wird. Der Betreiber hat vielmehr noch ein bis zwei Kalibrierintervalle Zeit, um eine Ersatzsonde einzubauen, da rechtzeitig erkannt wird, daß die alte Sonde aus dem Grenzbereich herausläuft.

In der Fig. 6 sind die Abfragepunkte durch Kreuze dargestellt. Sind die Kalibrierintervalle konstant, so muß die Zeit nicht erfaßt werden. Werden nicht äquidistante Kalibrierinvertalle genommen, muß der Mikrocomputer in der Lage sein, auch Zeitintervalle zu erfassen.

Die Kalibriereinrichtung nach Fig. 4 gestattet es auch, Zeit- und Driftverhalten auszugleichen, die mit einer Veränderung der Kennlinie einhergehen. Es gibt Sonden, deren Kennlinie sich nicht linear, wie dies in Fig. 2 gezeigt ist, verändern, sondern deren Kennlinie ein gekrümmtes Verhalten aufweist. Ist dieses gekrümmte Verhalten in Abhängigkeit vom Grenzstromsondensollwert bekannt, ist es möglich, im Speicher des Mikrocomputers eine Kennlinienschar abzulegen, wobei eine Kennlinie in Abhängigkeit vom Grenzstromsondensollwert herausgegriffen wird und die Regelung nach dieser Kennlinie erfolgt. Weiterhin ist es mit der Schaltungsanordnung nach Fig. 4 möglich, die Grenzstromsonde als solche zu überprüfen. Die Sonde erfährt nämlich teilweise Veränderungen, die die Sonde aus ihrem Grenzstromverhalten herausführt und sie empfindlicher gegenüber anderen Einflußgrößen wie beispielsweise die Sondentemperatur macht. Ein solches Verhalten ist in Fig. 5 dargestellt. In einer durchgezogenen Linie ist im stark vergrößerten Maßstab die Kennlinie einer einwandfreien Sonde dargestellt, während die gestrichelte Kennlinie eine beschädigte Grenzstromsonde kennzeichnet. Wird nun die Spannung der Gleichspannungsquelle 20 stetig oder schrittweise geändert und dabei der Grenzstrom überprüft, ist es möglich, aufgrund der dabei auftretenden Stromänderung einen Fehler in der Sonde zu erkennen. Diese Abfrage kann durch den Mikrocomputer ohne Schwierigkeiten vorgenommen werden. Durch die rechnerische Ermittlung der Neigung, die sich aus der Stromdifferenz durch die Spannungsdifferenz der Sonde bestimmt, kann nach dem Überschreiten eines bestimmten Neigungswertes ein Sondenschaden festgestellt werden. In Fig. 5 ist als Beispiel eine Zweipunktkontrolle angedeutet, wobei bei den Spannungswerten a und b der Sondenstrom bestimmt wird. Liegt dabei der Sondenstrom innerhalb eines Toleranzfeldes, so ist die Sonde in Ordnung, fällt die Kennlinie einseitig oder auf beiden Seiten aus dem Toleranzfeld heraus, so ist die Sonde schadhaft. Ebenso ist eine Überprüfung dadurch möglich, daß der Sondengleichspannungsquelle 20 eine dreieckige oder andere periodisch verlaufende Funktion überlagert wird und der Grenzstrom ebenfalls gewisse vorgegebene Grenzen nicht überschreitet. Um dynamische Effekte gering zu halten, ist es vorteilhaft, die Frequenz sehr klein zu wählen, da ansonsten das Grenzintervall erweitert werden muß.

Die aufgezeigten Meßverfahren sind nicht auf Grenzstromsonden in Heizanlagen oder an Verbrennungsmotoren von Kraftfahrzeugen beschränkt. Sie sind überall dort verwendbar, wo ohne große Umstände Gas mit bekanntem oder konstantem Sauerstoffgehalt an die Meßwertstelle gebracht werden kann. Durch Korrektur der Regelkreisparameter arbeitet die Regelung immer in der besten Parametereinstellung. Weiterhin ist es mit diesem Verfahren möglich, auch Grenzstromsonden zu verwenden, die in ihren Daten nicht exakt identisch sind, ohne daß das Regelverhalten darunter leidet.

## Patentansprüche

1. Verfahren zur Überwachung von Sauerstoff-Grenzstromsonden, wobei die Sauerstoff-Grenzstromsonde (2) mit einer elektrischen Spannung beaufschlagbar ist, dadurch gekennzeichnet, daß die Überwachung der Sauerstoff-Grenzstromsonde bei einer Spülung derselben mit einem Normgas erfolgt, wobei die der Sauerstoff-Grenzstromsonde (2) beaufschlagte elektrische Spannung um einen Mittelwert variiert wird, die resultierende Stromänderung der Sauerstoff-Grenzstromsonde (2) ermittelt wird und bei Überschreiten eines vorgegebenen Grenzwertes der auf die Spannungsdifferenz bezogenen Stromdifferenz auf einen Fehler der Sauerstoff-Grenzstromsonde (2) erkannt wird.

2. Verfahren nach Anspruch 1, dadurch gekennzeichnet, daß die Variation der elektrischen Spannung stufenweise erfolgt.

3. Verfahren nach Anspruch 1, dadurch gekennzeichnet, daß die Variation der elektrischen Spannung mit einer Frequenz von < 1 Hz erfolgt.

4. Vorrichtung zur Überwachung von Sauerstoff-Grenzstromsonden zur Durchführung des Verfahrens nach den Ansprüchen 1 bis 3, dadurch gekennzeichnet, daß eine Spannungsquelle zur Beaufschlagung der Sauerstoff-Grenzstromsonde (2) mit einer elektrischen Spannung vorhanden ist, welche während der Spülung der Sauerstoff-Grenzstromsonde (2) mit Normgas durch eine Rechenvorrichtung (22) variierbar ist, daß die dabei auftretenden Stromwerte von der Rechenvorrichtung (22) erfaßbar sind, und daß die Rechenvorrichtung (22) derart ausgebildet ist, daß die auf die Spannungsdifferenzen bezogenen Stromdifferenzen mit in Speichern (23) der Rechenvorrichtung (22) abgelegten Werten vergleichbar sind.

5. Vorrichtung nach Anspruch 4, dadurch gekennzeichnet, daß die Rechenvorrichtung (22) derart ausgebildet ist, daß ein Fehlalarm oder eine Abschaltung der Sauerstoff-Grenzstromsonde (2) erfolgt, wenn vorgegebene Toleranzen für auf Spannungsvariationen bezogene Stromänderungen überschritten werden.

## Claims

1. Method for monitoring oxygen maximum diffusion current probes, an electrical voltage being applied to the oxygen maximum diffusion current probe (2), characterized in that the monitoring of the oxygen maximum diffusion current probe is carried out with the latter being flushed with a standard gas, the electrical voltage applied to the oxygen maximum diffusion current probe (2) being varied around a mean, the resulting current variation of the oxygen maximum diffusion current probe (2) being determined and a fault in the oxygen maximum diffusion current probe (2) being detected if a specified limiting value of the current difference, which is related to the voltage difference, is exceeded.

2. Method according to Claim 1, characterized in that the variation of the electrical voltage takes place stepwise.

3. Method according to Claim 1, characterized in that the variation of the electrical voltage takes place at a frequency of < 1 Hz.

4. Device for monitoring oxygen maximum diffusion current probes to carry out the method according to Claims 1 to 3, characterized in that a voltage source is present for applying an electrical voltage to the oxygen maximum diffusion current probe (2), which source can be varied by a computer device (22) during the flushing of the oxygen maximum diffusion current probe (2) with standard gas, in that the current values encountered in this process can be assessed by the computer device (22), and in that the computer device (22) is constructed in a manner such that current differences related to the voltage differences can be compared with values stored in the memories (23) of the computer device (22).

5. Device according to Claim 4, characterized in that the computer device (22) is constructed in a manner such that a fault alarm is indicated or the oxygen maximum diffusion current probe (2) is switched off if specified tolerances for current variations related to voltage variations are exceeded.

## Revendications

1. Procédé de surveillance des sondes à oxygène à intensité limite, dans lequel une tension électrique peut intervenir sur la sonde à oxygène à intensité limite (2), caractérisé en ce que la surveillance de la sonde à oxygène à intensité limite se fait lors d'un balayage de cette sonde avec un gaz normalisé, étant précisé que l'on fait varier, autour d'une valeur moyenne, la tension électrique qui intervient sur la sonde à oxygène à intensité limite (2), que l'on établit la modification résultante de l'intensité de la sonde à oxygène à intensité limite (2) et que, en cas de dépassement, par valleur supérieure, d'une valleur limite, prédéterminée, de la différence d'intensité rapportée à la différence de tension, on en conclut à un défaut de la sonde à oxygène à intensité limite (2).

2. Procédé selon la revendication 1, caractérisé en ce que la variation de la tension électrique se fait par pas.

3. Procédé selon la revendication 1, caractérisé en ce que la variation de la tension électrique se fait avec une fréquence inférieure à 1 Hz.

4. Dispositif pour la surveillance des sondes à oxygène à intensité limite, pour l'exécution du procédé selon les revendications 1 à 3, caractérisé en ce qu'une source de courant utilisée pour intervenir sur la sonde à oxygène à intensité limite (2) présente une tension électrique qu'une installation de calcul (22) peut faire varier pendant le balayage de la sonde à oxygène à intensité limite (2) par le gaz normalisé; en ce que l'installation de calcul (22) peut saisir les valeurs de l'intensité qui apparaissent alors; et en ce que l'installation de calcul (22) est conçue de façon telle que les différences d'intensité, rapportées aux différences de tension puissent être comparées avec des valeurs entrées dans des mémoires (23) de l'installation de calcul (22).

5. Dispositif selon la revendication 4, caractérisé en ce que l'installation de calcul (22) est conçue de façon telle qu'il se produise une alarme de signalisation de défaut ou une mise hors circuit de la sonde à oxygène à valeur limite (2), lorsque des tolérances prédéterminées sont dépassées pour les modifications d'lintensité rapportées aux modifications de tension.

Fig. 1

Fig. 2

Fig. 3

Fig. 4

Fig. 5

Fig. 6